(19) Europäisches Patentamt
European Patent Office
Office européen des brevets

(11) Veröffentlichungsnummer : **0 275 892 B1**

(12) **EUROPÄISCHE PATENTSCHRIFT**

(45) Veröffentlichungstag der Patentschrift : **17.04.91 Patentblatt 91/16**

(51) Int. Cl.[5] : **C07C 45/38,** C07C 47/198, C07C 47/277

(21) Anmeldenummer : **88100280.2**

(22) Anmeldetag : **12.01.88**

(54) **Verfahren zur Oxydehydrierung von Polyglykolmonoäthern zu den entsprechenden Aldehyden.**

(30) Priorität : **17.01.87 DE 3701304**

(43) Veröffentlichungstag der Anmeldung : **27.07.88 Patentblatt 88/30**

(45) Bekanntmachung des Hinweises auf die Patenterteilung : **17.04.91 Patentblatt 91/16**

(84) Benannte Vertragsstaaten : **BE CH DE ES FR GB IT LI NL SE**

(56) Entgegenhaltungen :
**DE-A- 2 902 805**
**US-A- 2 307 934**

(73) Patentinhaber : **HOECHST AKTIENGESELLSCHAFT**
**Postfach 80 03 20**
**W-6230 Frankfurt am Main 80 (DE)**

(72) Erfinder : **Driscoll, Robert Kenneth, Dr.**
**Heilmannstrasse 43**
**W-6000 Frankfurt am Main 50 (DE)**
Erfinder : **Leupold, Ernst Ingo, Dr.**
**Am Zäunefeld 15**
**W-6392 Neu-Anspach (DE)**

**EP 0 275 892 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Verfahren zur Oxydehydrierung von Polyglykolmonoäthern zu den entsprechenden Aldehyden.

Die Erfindung betrifft ein Verfahren zur katalytischen Oxydehydrierung von Polyglykolmonoäthern der allgemeinen Formel $RO(CH_2CH_2O)_nCH_2CH_2OH$ (n = 1 bis 3) zu den entsprechenden Aldehyden in der Gasphase:

$$RO(CH_2CH_2O)_nCH_2CH_2OH + \frac{1}{2}O_2 \xrightarrow{Kat.} RO(CH_2CH_2O)_nCH_2CHO + H_2O$$

Aus US-PS 3 462 495 ist bereits bekannt, daß Polyglykolmonoäther sich in Abwesenheit von Sauerstoff an einem Calcium-Nickel-Phosphat-Katalysator dehydrieren lassen. Dabei werden Monoglykolmonoäther zum entsprechenden Aldehyd umgewandelt, während Polyglykolmonoäther (n = 1 oder 2) zu deren einfachsten Aldehyden ($ROCH_2CHO$) abgebaut werden (Seite 1, Zeile 24-28 und Beispiel 2).

In der US-PS 2 307 934 wird die Oxydehydrierung von Alkoholen der allgemeinen Formel $ROC(R_1R_2)OC_nH_{2n}OH$ (R = Alkyl ; $R_1$ und $R_2$ = H oder Alkyl ; n größer als 1) bei etwa 400°C an Silbernetzen beschrieben. Diese Alkohole unterscheiden sich von den Polyglykolmonoäthern dadurch, daß sie eine O-C-O Funktion (Acetalfunktion) enthalten. Die instabileren Alkohole werden vorzugsweise in einem $O_2$-Unterschuß oxydehydriert, wobei jedoch höchstens 30% Umsatz und eine dementsprechende niedrige Ausbeute erreichbar ist. Bei den stabileren Alkoholen, wie Methoxymethoxyäthanol, wird zwar ein $O_2$-Überschuß verwendet (Beispiel 1, Anspruch 9), die beschriebenen Ausbeuten liegen jedoch auch hier unterhalb 35%.

Es wurde nun gefunden, daß hohe Ausbeuten an Aldehyden der allgemeinen Formel $RO(CH_2CH_2O)_nCH_2CHO$ (n = 1, 2 oder 3) erreicht werden durch Oxydehydrierung der entsprechenden Polyglykolmonoäther in der Gasphase an Katalysatoren, die Silber enthalten oder aus Silber bestehen.

Nach US-PS 3 462 495 werden Polyglykolmonoäther bei der Dehydrierung zu deren einfachsten Aldehyden ($ROCH_2CHO$) abgebaut. Die in der US-PS 2 307 934 beschriebene Oxydehydrierung von ähnlichen Alkoholen an Silbernetzen ergibt nur schlechte Ausbeuten. Es ist daher sehr überraschend, daß sich Polyglykolmonoäther an silberhaltigen Katalysatoren zu den entsprechenden Aldehyden in guter Ausbeute oxydehydrieren lassen.

Gegenstand der vorliegenden Erfindung ist daher ein Verfahren zur katalytischen Oxydehydrierung von Polyglykolmonoäthern der allgemeinen Formel $RO(CH_2CH_2O)_nCH_2CH_2OH$ zu den entsprechenden Aldehyden der Formel $RO(CH_2CH_2O)_nCH_2CHO$ in der Gasphase, wobei n = 1, 2 oder 3 ist und R ein Phenyl-, Benzyl- oder ein Alkylrest mit 1 bis 8 C-Atomen ist, das dadurch gekennzeichnet ist, daß der Katalysator Silber enthält oder aus Silber besteht. Vorzugsweise ist n = 1 oder 2. Der Alkylrest R kann geradkettig oder verzweigt sein und hat vorzugsweise 1 bis 4 C-Atome, ist also z.B. der Methyl-, Äthyl-, n- oder isoPropyl- oder der n-, sek.-, tert.- oder iso-Butylrest. Besonders bevorzugt als Ausgangsmaterialien sind die $C_1$-$C_4$-Äther des Diäthylenglykols.

Die als Ausgangsmaterial dienenden Polyglykolmonoäther sind leicht zugänglich, z.B. durch katalytische Reaktion von Äthylenoxid mit einem Alkohol (Weissermel/Arpe, Industrielle Organische Chemie. S. 133, Verlag Chemie, 1978).

Erfindungsgemäß werden die Polyglykolmonoether in Dampfform zusammen mit Sauerstoff über den Katalysator geleitet. In vielen Fällen hat es sich als zweckmäßig erwiesen, unter Zumischung eines Inertgases zu arbeiten. Als Inertgas kommen z.B. Stickstoff, Edelgase und Kohlendioxid infrage. Falls man Luft verwendet, erfüllt diese gleichzeitig die Funktion des Inertgases und der $O_2$-Quelle. In den meisten Fällen ist die Zufuhr von Wasserdampf in den Reaktionsraum vorteilhaft.

Bei dem erfindungsgemäßen Verfahren benötigt man pro Mol Polyglykolmonoäther 0,5 Mol Sauerstoff. Man kann jedoch die eingesetzte Menge Sauerstoff in weiten Grenzen variieren, z.B. im Bereich von 0,1 bis 20 Mol, vorzugsweise 0,5 bis 2,5 Mol. Die Menge Inertgas kann z.B. 0 bis 80 Mol und die Menge Wasser 0 bis 10 Mol, vorzugsweise 0 bis 3 Mol betragen.

Das als Katalysator wirkende Silber wird entweder allein, z.B. als Silbernetz, oder vorzugsweise auf einem geeigneten Träger eingesetzt. Die BET-Oberfläche (Brunauer-Emmett-Teller-Oberfläche) des Trägers soll in diesem Fall unterhalb 100 $m^2g^{-1}$ liegen, vorzugsweise unterhalb 30 $m^2g^{-1}$. Viele Materialien können als Träger fungieren. Vorzugsweise verwendet man Aluminiumoxid, Siliciumdioxid, Aluminiumsilicat, Magnesiumsilicat, Calciumsilicat, Aluminiummagnesiumsilicat, Titandioxid oder deren Gemische. Besonders bevorzugt sind Magnesiumsilicat, Aluminiumsilicat und Aluminiummagnesiumsilicat.

Das Trägermaterial kann in vielen verschiedenen geometrischen Formen eingesetzt werden. Besonders geeignete Formen sind z.B. Kugeln oder die in der DE-OS 3 401 115 beschriebenen Monolithstrukturen, die als "statische Mischer" bekannt sind. Diese Monolithstrukturen sind in den Abbildungen 1 und 2 wiedergegeben. Solche Monolithe bestehen aus parallel zur Reaktorachse angeordneten, aufeinander liegenden gewellten Schichten (also etwa wie Wellblech geformt), wobei Wellentäler in aufeinander folgenden Schichten alternierend einen Winkel von $\alpha$ und $-\alpha$ mit der Reaktorachse bilden ($\alpha$ = 20-70°). Die durch die Wellentäler und Wellenkämme der aufeinanderliegenden Schichten gebildeten Kanäle haben den Querschnitt eines Dreiecks mit stark abgerundeten Ecken. Zwischen den aufeinander liegenden gewellten Schichten existiert an jeder Berührungsstelle eine feste Verbindung, so daß jedes Monolithelement eine einzige mit Kanälen durchzogene Hohlraumstruktur darstellt. Im allgemeinen werden die Monolithe in Form von mehreren aufeinander gestapelten Zylindern eingesetzt. Die beschriebene Struktur stellt dabei automatisch sicher, daß die Kanäle der aufeinander gestapelten Zylinder so zueinander passen, daß das eingesetzte Gas vom Eingang des Reaktorrohrs zum Ausgang fließen kann.

Solche Monolithkatalysatoren eignen sich z.B. für den Einsatz in größeren Reaktorrohren, u.a. weil der Druckverlust in der Katalysatorschüttung bei kürzeren Verweilzeiten sehr niedrig ist und weil die Reaktionswärme effektiver auf die gekühlte Reaktorwand übertragen wird.

Silberkatalysatoren, die zusätzlich Phosphor enthalten, sind ebenfalls für die erfindungsgemäße Oxydehydrierung geeignet. Der Zusatz von Phosphor bewirkt zwar manchmal eine geringfügige Abnahme der Ausbeute des erwünschten Aldehyds, vermindert jedoch gleichzeitig die unerwünschte Totaloxidation, mit dem Vorteil einer geringeren Wärmeentwicklung.

Wenn man einen Trägermaterial enthaltenden Silberkatalysator benutzt, kann die Menge an Silber bzw. an Phosphor auf dem Träger in weiten Grenzen schwanken. Die Silbermenge beträgt im allgemeinen 0,01 bis 50 Gew.-%, vorzugsweise 0,1 bis 20 Gew.-%, und die Phosphormenge 0 bis 25 Gew.-%, vorzugsweise 0 bis 15 Gew.-%, jeweils bezogen auf die Gesamtmasse des Katalysators. Dabei beträgt das Atomverhältnis P : Ag im allgemeinen 0 bis 4, vorzugsweise 0 bis 2.

Ag wird entweder in metallischer Form oder in Form einer Verbindung, z.B. als Nitrat, Phosphat, Chlorat, Acetat, Lactat, Propionat oder Tartrat in den Katalysator eingebracht. P wird entweder als Element oder (vorzugsweise) in Form seiner Verbindung, z.B. als Phosphat oder als eine der Phosphorsäuren eingesetzt.

Silber und ggf. Phosphor werden zweckmäßig in Form einer Lösung auf den Träger aufgebracht ; dann wird das Lösemittel abgedampft und der Katalysator getrocknet. Als Lösemittel verwendet man im allgemeinen Wasser, Salzsäure, Salpetersäure, Alkalilaugen oder Ammoniaklösung.

Es hat sich als vorteilhaft erwiesen, vor dem Einleiten des Polyglykolmonoäthers in die Reaktionszone ein oxidierendes Gas, insbesondere Sauerstoff,der als solcher oder in Form von Luft angewendet werden kann, bei Temperaturen von 100 bis 800°C, insbesondere 200 bis 600°C, über den Katalysator zu leiten.

Das erfindungsgemäße Verfahren wird im allgemeinen bei Temperaturen zwischen 300 und 700°C, vorzugsweise zwischen 320 und 520°C durchgeführt. Die Verweilzeit liegt vorzugsweise zwischen 0,01 und 10 Sekunden, insbesondere jedoch zwischen 0,05 und 1,5 Sekunden. Im allgemeinen werden mit zunehmender Reaktionstemperatur kürzere Verweilzeiten gewählt.

Das erfindungsgemäße Verfahren wird bevorzugt bei Atmosphärendruck durchgeführt, jedoch können auch verminderte oder erhöhte Drucke angewendet werden (0,01 bis 100 bar).

Im einzelnen geht man so vor, daß der Polyglykolmonoether, bzw. sein Gemisch mit Wasser, aus einer Dosiervorrichtung in eine Verdampfungszone und das entstandene Gas dann durch ein von außen beheiztes und mit dem Katalysator gefülltes Reaktionsrohr geleitet wird. In der Verdampfungszone erfolgt gegebenenfalls die Vermischung mit dem Inertgas und dem Sauerstoff oder dem sauerstoffhaltigen Gas ; es hat sich als vorteilhaft erwiesen, diese Gase vor der Vermischung auf die Reaktionstemperatur vorzuheizen. Die Reaktionsprodukte werden nach Verlassen des Reaktors zur Abtrennung der kondensierbaren Anteile gekühlt.

Die Verfahrensprodukte sind wertvolle Zwischenprodukte, z.B. für die Herstellung von synthetischen Harzen (K.F. Beal et al., J. Polymer Sci., 1 (6), S. 540 (1946).

Die nachfolgenden Beispiele sollen die Erfindung näher erläutern.

**Beispiele**

1) Zur Herstellung des Katalysators wurden 50 g eines Aluminiumsilicat-Trägers (BET-Oberfläche 1 $m^2/g$) mit einer Lösung von 2,5 g $Ag_3PO_4$ in 17,5 ml konzentriertem Ammoniak getränkt und das Lösemittel auf dem Dampfbad abgedampft. Der Katalysator wurde bei 110°C getrocknet. Anschließend wurde in gleicher Weise eine Lösung von 1,19 g kristalliner $H_3PO_4$ in 13,8 ml Wasser aufgebracht und der Katalysator erneut bei 110°C getrocknet. Der Katalysator enthielt 3,87 Gew.-% Ag und 1,13 Gew.-% P, was einem Ag : P-Atomverhältnis von 1 : 1 entspricht.

10 ml (etwa 10.9 g) dieses Katalysators wurden in ein von außen beheiztes Glasreaktorrohr gefüllt und wie folgt kalziniert : Der Katalysator wurde innerhalb von 6 Std. langsam in einem Gasstrom aus 7,3 Nl/h Sauerstoff und 56 Nl/h Stickstoff auf 450°C erhitzt. Anschließend wurden 5,4 g/h eines Gemisches aus Diäthylenglykolmonomethyläther (92,6 Gew.-%) und Wasser (7,4 Gew.-%) mit Hilfe einer Kolbenspritze

über eine Verdampfungszone in ein senkrecht angeordnetes Reaktionsrohr (Länge : 15 cm, Durchmesser: 2 cm) eingeleitet. Gleichzeitig wurden 58 Nl/h Stickstoff und 1,4 Nl/h Sauerstoff zugeführt, die beide vorher auf 400°C aufgeheizt worden waren. Die Reaktionstemperatur von 400°C wurde mit Hilfe eines beweglichen Thermoelements im Inneren des Reaktors gemessen. Die Reaktionsprodukte wurden in einer Kühlfalle bei – 70°C kondensiert und mit gaschromatographischen Methoden analysiert.

Nach einer Anlaufzeit von 1 Stunde zur Einstellung konstanter Betriebsbedingungen wurde der eigentliche Versuch über einen Zeitraum von 2 Stunden durchgeführt. Als Nebenprodukte wurden Äthylenglykolmonomethyläther, Methoxyacetaldehyd, CO, $CO_2$, HCHO und Methanol gefunden. Als Ergebnis des zweistündigen Versuches wurden 59,2 mmol 3,6-Dioxaheptanal ($CH_3OCH_2CH_2OCH_2CHO$) und 8,7 mmol Diäthylenglykolmonomethyläther im Kondensat gefunden, was 71,1% Ausbeute bei 89,6% Umsatz entspricht. Der Aldehyd wurde mittels Vakuumdestillation aus dem Kondensat isoliert. Der Siedepunkt betrug 87°C bei 60 mbar.

2) Zur Herstellung des Katalysators wurden 50 g eines Magnesiumsilicat-Trägers (BET-Oberfläche kleiner als 1 $m^2/g$) mit einer Lösung von 3,1 g $AgNO_3$ in 12,5 ml Wasser getränkt und das Lösemittel auf dem Dampfbad abgedampft. Der Katalysator wurde anschließend in gleicher Weise wie im Beispiel 1 getrocknet und kalziniert. Danach lag ein großer Teil des Silbers in metallischer bzw. oxydischer Form vor. Der Katalysator enthielt 3,9 Gew.-% Ag.

Analog zu Beispiel 1 wurden in die dort beschriebene Apparatur 5,0 g/h Diäthylenglykolmonomethyläther, 0,4 g/h Wasser, 31 Nl/h Stickstoff und 0,94 Nl/h Sauerstoff über 10 ml des Katalysators (etwa 8,5 g) geleitet. Die Reaktionstemperatur betrug 435°C. Als Ergebnis eines zweistündigen Versuches wurden 63,6 mmol 3,6-Dioxaheptanal und 3,4 mmol Diäthylenglykolmonomethyläther im Kondensat gefunden, was 76,3% Ausbeute bei 95,9% Umsatz entspricht.

3) 10 ml des im Beispiel 2 beschriebenen Katalysators wurden verwendet. Analog zu Beispiel 1 wurden in die dort beschriebene Apparatur 6,9 g/h Diäthylenglykolmono-n-butyläther, 0,4 g/h Wasser, 31 Nl/h Stickstoff und 0,94 Nl/h Sauerstoff eingeleitet. Die Reaktionstemperatur betrug 450°C. Als Ergebnis eines zweistündigen Versuches wurden 55,7 mmol 3,6-Dioxadecanal und 4,5 mmol Diäthylenglykolmono-n-butyläther im Kondensat gefunden, was 65,4% Ausbeute an Aldehyd bei 94,7% Umsatz entspricht. Der Aldehyd wurde mittels Vakuumdestillation aus dem Kondensat isoliert. Der Siedepunkt betrug 92°C bei 20 mbar.

## Ansprüche

1. Verfahren zur katalytischen Oxydehydrierung von Polyglykolmonoäthern der allgemeinen Formel $RO(CH_2CH_2O)_nCH_2CH_2OH$ zu den entsprechenden Aldehyden der Formel $RO(CH_2CH_2O)_nCH_2CHO$ in der Gasphase, wobei n = 1, 2 oder 3 ist und R ein Phenyl-, Benzyl- oder ein Alkylrest mit 1 bis 8 C-Atomen ist, dadurch gekennzeichnet, daß der Katalysator Silber enthält oder aus Silber besteht.

2. Verfahren nach Anspruch 1, dadurch gekennzeichnet, daß der Katalysator zusätzlich Phosphor enthält, wobei der Phosphorgehalt zweckmäßig höchstens 25, vorzugsweise höchstens 15 Gew.-%, bezogen auf das Gesamtgewicht des Katalysators, und das Atomverhältnis P : Ag im allgemeinen bis zu 4, vorzugsweise bis zu 2 beträgt.

3. Verfahren nach Anspruch 1 oder 2, dadurch gekennzeichnet, daß der Katalysator Silber auf einem Trägermaterial mit einer BET-Oberfläche unterhalb von 100 $m^2g^{-1}$, vorzugsweise unterhalb 30 $m^2g^{-1}$ enthält.

4. Verfahren nach Anspruch 3, dadurch gekennzeichnet, daß Aluminiumoxid, Siliciumdioxid, Aluminiumsilicat, Magnesiumsilicat, Calciumsilicat, Aluminiummagnesiumsilicat, Titandioxid oder deren Gemische als Trägermaterial verwendet werden.

5. Verfahren nach mindestens einem der Ansprüche 1 bis 4, dadurch gekennzeichnet, daß Polyglykolmonoäther mit n = 1 oder 2 eingesetzt werden, wobei R vorzugsweise $C_1$-$C_4$-Alkyl ist.

6. Verfahren nach mindestens einem der Ansprüche 1 bis 5, dadurch gekennzeichnet, daß in Gegenwart eines Inertgases und/oder von Wasserdampf gearbeitet wird.

7. Verfahren nach mindestens einem der Ansprüche 1 bis 6, dadurch gekennzeichnet, daß die Reaktionstemperatur zwischen 300 und 700°C, vorzugsweise zwischen 320 und 520°C liegt.

8. Verfahren nach mindestens einem der Ansprüche 1 bis 7, dadurch gekennzeichnet, daß die Verweilzeit der Reaktionsteilnehmer zwischen 0,01 und 10, vorzugsweise 0,05 und 1,5 Sekunden liegt.

9. Verfahren nach mindestens einem der Ansprüche 1 bis 8, dadurch gekennzeichnet, daß bei einem Druck von 0,01 bis 100 bar, vorzugsweise bei Atmosphärendruck, gearbeitet wird.

10. Verfahren nach mindestens einem der Ansprüche 1 bis 9, dadurch gekennzeichnet, daß 0,1 bis 20 Mol, vorzugsweise 0,5 bis 2,5 Mol Sauerstoff je Mol Polyglykolmonoäther angewendet werden.

11. Verfahren nach mindestens einem der Ansprüche 1 bis 10, dadurch gekennzeichnet, daß der Katalysator vor der Reaktion mit einem oxydierenden Gas, insbesondere Luft, bei Temperaturen von 100 bis 800°C, vorzugsweise 200 bis 600°C, in Berührung gebracht wird.

## Claims

1. A process for the catalytical oxydehydrogenation of polyglycolmonoethers of the general formula $RO(CH_2CH_2O)_nCH_2CH_2OH$ to yield the corresponding aldehydes of the formula $RO(CH_2CH_2O)_nCH_2CHO$ in which formulae n is 1, 2 or 3 and R represents a phenyl, benzyl or an alkyl group having form 1 to 8 carbon atoms, which comprises using a catalyst containing silver or consisting of silver.

2. A process as claimed in claim 1, wherein the catalyst also contains phosphorus, the content of phosphorus suitably being at most 25%, preferably at most 15%, referred to the total weight of the catalyst, and wherein the atomic ratio P : Ag in the catalyst in general is up to 4, preferably up to 2.

3. A process as claimed in claim 1 or 2, wherein the catalyst comprises silver deposited on a carrier material having a BET-surface lower than 100 $m^2g^{-1}$, preferably lower than 30 $m^2g^{-1}$.

4. A process as claimed in claim 3, wherein as the carrier material there is used alumina, silica, aluminium silicate, magnesium silicate, calcium silicate, aluminium magnesium silicate, titanium dioxide or a mixture thereof.

5. A process as claimed in one or more of claims 1 to 4, wherein a polyglycolmonoether is used in which n is 1 or 2 and wherein R preferably is alkyl having from 1 to 4 carbon atoms.

6. A process as claimed in one or more of claims 1 to 5, which is carried out in the presence of a gas inert towards the reactants and/or of water vapor.

7. A process as claimed in one or more of claims 1 to 6, wherein the reaction temperature is between 300 and 700°C, preferably between 320 and 520°C.

8. A process as claimed in one or more of claims 1 to 7, wherein the residence time of the reactants is in the range of from 0.01 to 10 seconds, preferably in the range of from 0.05 to 1.5 seconds.

9. A process as claimed in one or more of claims 1 to 8, wherein the reaction is carried out at a pressure in the range of from 0.01 to 100 bar, preferably at ambient pressure

10. A process as claimed in one or more of claims 1 to 9, wherein about 0.1 to 20 mol, preferably 0.5 to 2,5 mol, of oxygen are applied for every mol of polyglycolmonoether

11. A process as claimed in one or more of claims 1 to 10, wherein the catalyst is contacted with an oxidizing gas, in particular with air, at a temperature in the range of from 100 to 800°C, preferably of from 200 to 600°C, before the oxydehydrogenation is started.

## Revendications

1. Procédé pour oxydéshydrogéner catalytiquement, en phase gazeuse, des mono-éthers de polyglycols de formule générale :

$$RO(CH_2CH_2O)_nCH_2CH_2OH$$

et les convertir ainsi en les aldéhydes correspondants de formule :

$$RO(CH_2CH_2O)_nCH_2CHO,$$

formules dans lesquelles n désigne un nombre égal à 1, à 2 ou à 3 et R représente un phényle, un benzyle ou un alkyle contenant de 1 à 8 atomes de carbone, procédé caractérisé en ce que le catalyseur contient de l'argent ou est constitué d'argent.

2. Procédé selon la revendication 1 caractérisé en ce que le catalyseur contient en outre du phosphore, la teneur en phosphore étant avantageusement d'au plus 25, de préférence d'au plus 15% en poids, par rapport au poids total du catalyseur, et le rapport atomique P : Ag étant en général d'au plus 4, de préférence d'au plus 2.

3. Procédé selon l'une des revendications 1 et 2, caractérisé en ce que le catalyseur contient de l'argent sur une matière support ayant une surface BET inférieure à 100 $m^2g^{-1}$, de préférence inférieure à 30 $m^2g^{-1}$.

4. Procédé selon la revendication 3 caractérisé en ce qu'on utilise, comme matière support, de l'oxyde d'aluminium, du dioxyde de silicium, du silicate d'aluminium, du silicate de magnésium, du silicate de calcium, du silicate d'aluminium et de magnésium, du dioxyde de titane ou des mélanges de ces composés.

5. Procédé selon l'une quelconque des revendications 1 à 4, caractérisé en ce qu'on utilise des mono-éthers de polyglycols dans lesquels n est égal à 1 ou 2 et dans lesquels R représente de préférence un radical alkyle en $C_1$-$C_4$.

6. Procédé selon l'une quelconque des revendications 1 à 5, caractérisé en ce qu'on opère en présence d'un gaz inerte et/ou de vapeur d'eau.

7. Procédé selon l'une quelconque des revendications 1 à 6, caractérisé en ce que la température réactionnelle est comprise entre 300 et 700°C, de préférence entre 320 et 520°C.

8. Procédé selon l'une quelconque des revendications 1 à 7 caractérisé en ce que le temps de séjour des corps participants à la réaction est compris entre 0,01 et 10 secondes, de préférence entre 0,05 et 1,5 secondes.

9. Procédé selon l'une quelconque des revendications 1 à 8, caractérisé en ce qu'on opère sous une pression de 0,01 à 100 bar, de préférence sous la pression atmosphérique.

10. Procédé selon l'une quelconque des revendications 1 à 9, caractérisé en ce qu'on met en jeu de 0,1 à 20 mol, de préférence de 0,5 à 2,5 mol, d'oxygène par mole du mono-éther de polyglycol.

11. Procédé selon l'une quelconque des revendications 1 à 10, caractérisé en ce que le catalyseur est mis en contact, avant la réaction, avec un gaz oxydant, plus particulièrement avec de l'air, à des températures de 100 à 800°C, de préférence de 200 à 600°C.

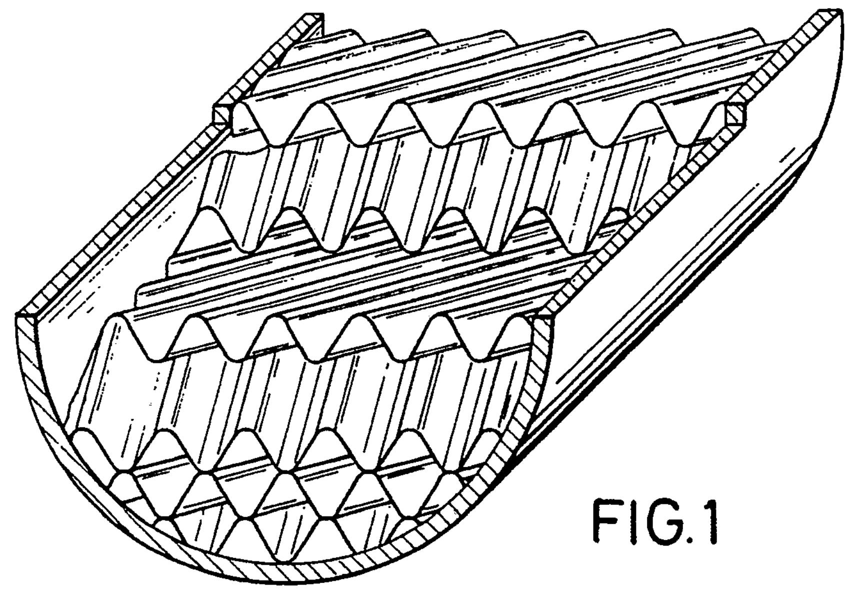

FIG.1

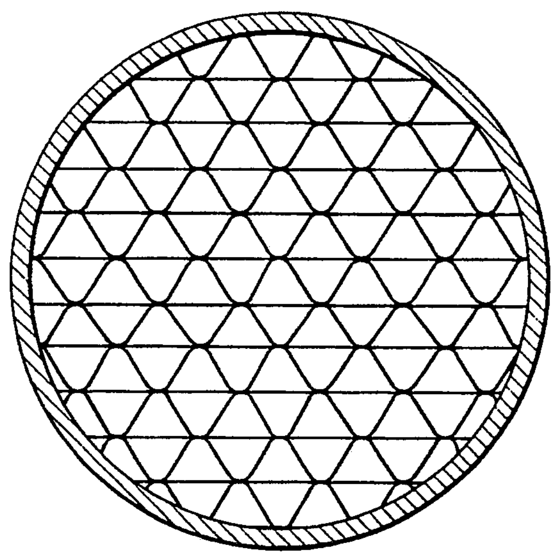

FIG.2